# EUROPÄISCHE PATENTSCHRIFT

(11) **EP 0 906 763 B1**
(45) Veröffentlichungstag und Bekanntmachung des Hinweises auf die Patenterteilung: **09.04.2003**
(21) Anmeldenummer: 98116583.0
(22) Anmeldetag: 02.09.1998
(51) Int. Cl.: A61L 2/22, A61L 2/20

(54) **Verfahren und Vorrichtung zum Entkeimen von Verpackungsmaterial**
Method and apparatus for sterilizing packaging material
Méthode et dispositif pour stériliser des matériaux d'emballage

(30) Priorität: 27.09.1997 DE 19742822
(43) Veröffentlichungstag der Anmeldung: 07.04.1999
(73) Patentinhaber: SIG Combibloc GmbH & Co. KG, 52441 Linnich (DE)
(72) Erfinder: Berger, Jörg, Dr., 52428 Jülich (DE); Offermanns, Guido, 52078 Aachen (DE)
(74) Vertreter: Cohausz & Florack

(56) Entgegenhaltungen:
- EP-A- 0 635 273
- DE-A- 2 703 524
- DE-A- 3 540 161
- GB-A- 2 199 245

## Beschreibung

Die Erfindung betrifft ein Verfahren zum Entkeimen von Verpackungsmaterial, insbesondere von Mehrschichtverbundverpackungen, mittels eines Wasserstoffperoxid enthaltenden Entkeimungsmittels, bei dem das Entkeimungsmittel zerstäubt und mit Druckluft gemischt, das Wasserstoffperoxid-Luft-Aerosol in einem überhitzten Wasserdampfstrom verdampft und anschließend im gasförmigen Zustand auf die zu entkeimende/n Fläche/n des Verpackungsmaterials geleitet wird. Weiterhin betrifft die Erfindung eine Vorrichtung zur Durchführung des Verfahrens sowie die Verwendung einer solchen Vorrichtung in einer Abfüllanlage für in Verpackungsbehälter einzubringendes Füllgut.

Ein derartiges Verfahren und eine entsprechende Vorrichtung sind aus der DE 35 40 161 C2 bekannt. Dabei wird zum Entkeimen von Verpackungsmaterial ein Wasserstoffperoxid-Luft-Aerosol taktweise durch eine Verdampfungseinrichtung gepreßt und anschließend im gasförmigen Zustand auf das zu entkeimende Verpackungsmaterial geleitet. Dabei werden Wasserstoffperoxid und Luft mit Hilfe einer Zweistoffdüse vernebelt und in die Verdampfungseinrichtung geleitet. Zum Erreichen eines guten Wärmeaustauschers sind in der Verdampfungseinrichtung Verwirbelungskörper (Drallkörper und Spiralfedern) eingesetzt, um möglichst das gesamte Wasserstoffperoxid-Luft-Aerosol mit der von außen beheizten Wandung der Verdampfungseinrichtung in Kontakt zu bringen.

Nachteilig bei diesem bekannten Verfahren bzw. der entsprechenden Vorrichtung und deren Verwendung ist die starke Abhängigkeit vom verwendeten Peroxid. Zur besseren Lagerhaltung wird Wasserstoffperoxid mit Stabilisatoren versetzt. Diese Stabilisatoren setzen sich als Rückstände an der Wandung und an den Verwirbelungskörpern der Verdampfungseinrichtung fest. Dadurch entsteht ein nicht unerheblicher Reinigungsaufwand, da zum ordnungsgemäßen Reinigen der Verdampfungseinrichtung die darin befindlichen Verwirbelungskörper ausgebaut werden müssen.

Durch die GB 2 199 245 A, von der die Erfindung ausgeht, ist ein Verfahren zum Entkeimen von Verpackungsmaterial bekannt, bei dem Wasserstoffperoxid verdampft und auf die zu entkeimenden Flächen des Verpackungsmaterials geleitet wird. Das Wasserstoffperoxid wird dazu in einem Strom von Wasserdampf oder feuchter, heißer Luft verdampft, wobei die Temperatur des Dampfes bzw. der feuchten, heißen Luft eingestellt werden kann. Nachteilig an einem derartigen Verfahren ist, daß ein vollständiges Verdampfen des Wasserstoffperoxids nicht sichergestellt ist.

Der Erfindung liegt daher die Aufgabe zugrunde, das eingangs genannte und zuvor näher beschriebene Verfahren sowie die entsprechende Vorrichtung zur Durchführung dieses Verfahrens so auszugestalten und weiterzubilden, daß ein vollständiges und optimales Verdampfen des Wasserstoffperoxids im Dampfstrom gewährleistet ist und somit Einbauten in der Misch- und Verdampferkammer überflüssig werden.

Verfahrensgemäß ist die Aufgabe dadurch gelöst, daß das Verdampfen des Wasserstoffperoxids bei Temperaturen im Bereich zwischen 500 und 600°C erfolgt.

Vorrichtungsmäßig ist die Aufgabe gelöst durch eine Überhitzungseinheit zur Überhitzung des Wasserdampfes und eine Misch- und Verdampferkammer mit einem Einlaß und einem Auslaß, wobei im Bereich des Einlasses eine Einsprühdüse zum Einsprühen des Wasserstoffperoxides angeordnet ist und wobei die Misch- und Verdampfungskammer einen sich konisch aufweitenden Durchmesser aufweist.

Im Unterschied zum bekannten Verfahren wird das Wasserstoffperoxid erfindungsgemäß in einem überhitzten Wasserdampfstrom verdampft. Dazu wird Wasserdampf überhitzt und auf eine Temperatur von 500 bis 600 °C gebracht. Fein vernebeltes Wasserstoffperoxid wird anschließend im Gleichstrom in den überhitzten Wasserdampf eingesprüht.

Das Einsprühen erfolgt dabei in der Misch- und Verdampferkammer mit einer konischen Aufweitung. Die Aufweitung entspricht dabei im wesentlichen bevorzugt der Strahlaufweitung der verwendeten Einsprühdüse. Die Durchmesser der Misch- und Verdampferkammer sind dabei so ausgelegt, daß sich im kleinsten Durchmesser das größte Tröpfchen im Schwebezustand befindet, während sich im größten Durchmesser das kleinste Tröpfchen im Schwebezustand befindet.

Gemäß einer weiteren Lehre der Erfindung kann die Misch- und Verdampferkammer eine doppelte Konizität aufweisen, so daß deren Einlaß- und Auslaßdurchmesser im wesentlichen gleich groß sind.

Es hat sich herausgestellt, daß mit dem erfindungsgemäßen Verfahren bzw. der erfindungsgemäßen Vorrichtung ein vollständiges Verdampfen des Wasserstoffperoxides möglich ist, ohne daß Einbauten in der Misch- und Verdampferkammer notwendig wären. Auf diese Weise läßt sich der Reinigungsaufwand drastisch reduzieren.

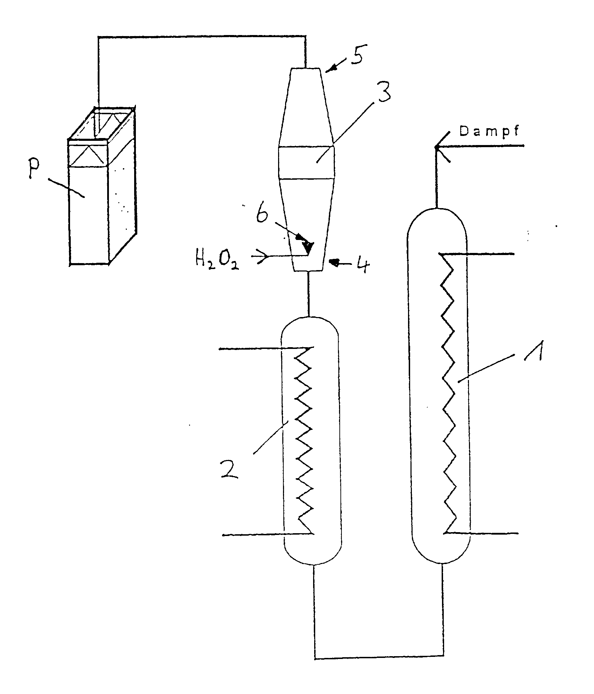

Die Erfindung wird nachfolgend anhand einer lediglich ein bevorzugtes Ausführungsbeispiel darstellenden Zeichnung näher erläutert. In der einzigen Figur gelangt Dampf in eine Überhitzungseinheit, die im dargestellten und insoweit bevorzugten Ausführungsbeispiel aus zwei Überhitzungsstufen 1 und 2 besteht. Der überhitzte Dampf wird dann einer Misch- und Verdampferkammer 3 zugeführt. Zweckmäßigerweise ist die zweite Überhitzungsstufe 2 unmittelbar vor dem Einlaß 4 der Misch- und Verdampferkammer 3 angeordnet.

Die dargestellte Misch- und Verdampferkammer 3 weitet sich zunächst von ihrem Einlaß 4 zur Mitte hin konisch auf und verjüngt sich anschließend wieder bis zu ihrem Auslaß 5. Im Bereich des Einlasses 4 ist eine Einsprühdüse 6 zur Zuführung des Wasserstoffperoxids vorgesehen, wobei zweckmäßigerweise die Konizität der sich aufweitenden Misch- und Verdampferkammer 3 der Strahlaufweitung der Einsprühdüse 6 entspricht. Durch diese Geometrie der Misch- und Verdampferkammer 3 wird erreicht, daß sich im kleinsten Durchmesser das größte Tröpfchen des Wasserstoffperoxids im Schwebezustand befindet, während sich im größten Durchmesser das kleinste Tröpfchen im Schwebezustand befindet. Dadurch wird eine gleichmäßige und vollständige Verdampfung des Wasserstoffperoxids erreicht.

Das auf diese Weise verdampfte Wasserstoffperoxid wird dann der (nicht dargestellten) Füllmaschine zugeführt und unmittelbar vor deren Befüllung in die zu entkeimenden Mehrschichtverbundpackung P geleitet.

Die vollständige und optimale Verdampfung wird dadurch erreicht, daß bei einer geringen Strömung in der Misch - und Verdampferkammer 3 und einer großen Tröpfchenoberfläche ein sehr guter Wärmeübergang gewährleistet ist. Durch die Verdampfung werden die Wasserstoffperoxidtröpfchen kleiner und damit leichter. Bedingt durch die Konizität der Misch- und Verdampferkammer 3 nimmt jedoch auch die Strömung ab, so daß bis zu vollständigen Verdampfung - etwa im mittleren Bereich der Misch- und Verdampferkammer 3 - ein gleichmäßiger Transport der Wasserstoffperoxidtröpfchen gewährleistet ist.

Nach dem der Verdampfungsvorgang beendet ist, etwa in der Mitte der Misch- und Verdampferkammer 3, nimmt der Durchmesser der Misch- und Verdampferkammer 3 zweckmäßigerweise wieder ab, um die Strömungsgeschwindigkeit wieder zu erhöhen.

## Patentansprüche

1. Verfahren zum Entkeimen von Verpackungsmaterial, insbesondere von Mehrschichtverbundverpackungen, mittels eines Wasserstoffperoxid enthaltenden Entkeimungsmittels, bei dem das Entkeimungsmittel zerstäubt und mit Druckluft gemischt, das Wasserstoffperoxid-Luft-Aerosol in einem überhitzten Wasserdampfstrom verdampft und anschließend im gasförmigen Zustand auf die zu entkeimende/n Fläche/n des Verpackungsmaterials geleitet wird,
**dadurch gekennzeichnet, daß** das Verdampfen des Wasserstoffperoxids bei Temperaturen im Bereich zwischen 500 und 600 °C erfolgt.

2. Verfahren nach Anspruch 1,
**dadurch gekennzeichnet, daß** die Überhitzung des Wasserdampfes mehrstufig erfolgt.

3. Verfahren nach Anspruch 2,
**dadurch gekennzeichnet, daß** die Überhitzung des Wasserdampfes zweistufig erfolgt.

4. Vorrichtung zur Durchführung des Verfahrens nach einem der Ansprüche 1 bis 3,
**gekennzeichnet durch** eine Überhitzungseinheit (1, 2) zur Überhitzung des Wasserdampfes und eine Misch- und Verdampferkammer (3) mit einem Einlaß (4) und einem Auslaß (5), wobei im Bereich des Einlasses (4) eine Einsprühdüse (6) zum Einsprühen des Wasserstoffperoxides angeordnet ist und wobei die Misch- und Verdampfungskammer (3) einen sich konisch aufweitenden Durchmesser aufweist.

5. Vorrichtung nach Anspruch 4,
**dadurch gekennzeichnet, daß** die Konizität der sich aufweitenden Misch- und Verdampferkammer (3) der Strahlaufweitung der Einsprühdüse (6) entspricht.

6. Vorrichtung nach Anspruch 4 oder 5,
**dadurch gekennzeichnet, daß** die Misch- und Verdampferkammer (3) eine doppelte Konizität besitzt, so daß Einlaß (4) und Auslaß (5) im wesentlichen den gleichen Durchmesser aufweisen.

7. Verwendung der Vorrichtung nach einem der Ansprüche 4 bis 6 in einer Abfüllanlage für in Verpackungsbehälter einzubringendes Füllgut mit einer Transportvorrichtung zur Förderung des Verpackungsmaterial bzw. der Verpackungsbehälter, mit einem Vorratsbehälter für das Entkeimungsmittel, einer Zerstäubereinrichtung und einem Dampferzeuger.

## Claims

1. A method for sterilizing packaging material, in particular, multi-layer composite packages, by means of a sterilizing agent that contains hydrogen peroxide, wherein the sterilizing agent is atomized and mixed with compressed air, and wherein the hydrogen peroxide/air aerosol is evaporated in a superheated stream of steam and subsequently conveyed onto the surface(s) of the packaging material to be sterilized in the gaseous state,
**characterized in that** the hydrogen peroxide is evaporated at temperatures between 500 and 600 °C.

2. The method according to Claim 1,
**characterized in**
**that** the superheating of the steam takes place in several stages.

3. The method according to Claim 2,
**characterized in**
**that** the superheating of the steam takes place in two stages.

4. A device for carrying out the method according to one of Claims 1-3,
**characterized by**
a superheating unit (1, 2) for superheating the steam, and by a mixing and evaporating chamber (3) that has an inlet (4) and an outlet (5), wherein a spray nozzle (6) for introducing the hydrogen peroxide is arranged in the region of the inlet (4), and wherein the mixing and evaporating chamber (3) has a conically widening diameter.

5. The device according to Claim 4,
**characterized in**
**that** the conicality of the widening mixing and evaporating chamber (3) corresponds to the widening of the jet produced by the spray nozzle (6).

6. The device according to Claim 4 or 5,
**characterized in**
**that** the mixing and evaporating chamber (3) has a dual conicality such that the inlet (4) and the outlet (5) essentially have the same diameter.

7. The use of the device according to one of Claims 4 to 6 in a filling system for material to be introduced into packaging containers, with a transport device for transporting the packaging material or the packaging containers, with a reservoir for the sterilizing agent, with an atomizing device and with a steam generator.

## Revendications

1. Procédé de stérilisation de matériaux d'emballage, en particulier d'emballages composites à plusieurs couches, au moyen d'un agent de stérilisation contenant du peroxyde d'hydrogène, dans lequel l'agent de stérilisation est atomisé et mélangé à de l'air comprimé, l'aérosol de peroxyde d'hydrogène et d'air est vaporisé dans un courant de vapeur d'eau surchauffée et est ensuite acheminé à l'état gazeux sur la ou les surfaces du matériau d'emballage à stériliser, **caractérisé en ce que** la vaporisation du peroxyde d'hydrogène se fait à des températures dans la plage comprise entre 500 et 600°C.

2. Procédé selon la revendication 1, **caractérisé en ce que** la surchauffe de la vapeur d'eau se fait en plusieurs étapes.

3. Procédé selon la revendication 2, **caractérisé en ce que** la surchauffe de la vapeur d'eau se fait en deux étapes.

4. Dispositif permettant de réaliser le procédé selon l'une quelconque des revendications 1 à 3, **caractérisé par** une unité de surchauffe (1, 2) pour surchauffer la vapeur d'eau et une chambre de mélange et de vaporisation (3) avec une entrée (4) et une sortie (5), où une buse d'injection (6) est ménagée dans la zone de l'entrée (4) pour injecter par dispersion le peroxyde d'hydrogène et où la chambre de mélange et de vaporisation (3) présente un diamètre s'évasant en forme conique.

5. Dispositif selon la revendication 4, **caractérisé en ce que** la conicité de la chambre de mélange et de vaporisation évasée (3) correspond à l'élargissement du faisceau de projection de la buse d'injection (6).

6. Dispositif selon les revendications 4 ou 5, **caractérisé en ce que** la chambre de mélange et de vaporisation (3) possède une double conicité si bien que l'entrée (4) et la sortie (5) présentent sensiblement le même diamètre.

7. Utilisation du dispositif selon l'une quelconque des revendications 4 à 6 dans une installation de remplissage pour des produits à introduire dans des récipients d'emballage avec un dispositif de transport pour acheminer le matériau d'emballage ou le récipient d'emballage, un réservoir de stockage pour l'agent de stérilisation, un dispositif d'atomisation et un générateur de vapeur.
